Europäisches Patentamt

European Patent Office

Office européen des brevets

(19)

(11) Numéro de publication: **0 185 586**
**A2**

## (12) DEMANDE DE BREVET EUROPEEN

(21) Numéro de dépôt: 85402402.3

(22) Date de dépôt: 04.12.85

(51) Int. Cl.⁴: **A61K 31/315**

(30) Priorité: 07.12.84 FR 8418756

(43) Date de publication de la demande:
25.06.86 Bulletin 86/26

(84) Etats contractants désignés:
BE CH DE GB IT LI

(71) Demandeur: SOCIETE CIVILE DE RECHERCHES ET
D'ETUDES THERAPEUTIQUES
33, Avenue Arouet
F-92160 Antony(FR)

(72) Inventeur: Suck, Catherine
10, Rue Emile Morel
F-92330 Sceaux(FR)
Inventeur: Chazot, Guy
31, Quai Jean-Jacques Rousseau
F-69350 La Mulatiere(FR)

(74) Mandataire: Combe, André et al
CABINET BEAU DE LOMENIE 55 rue d'Amsterdam
F-75008 Paris(FR)

(54) Médicaments à base de gluconate de zinc utiles pour le traitement des hyperprolactinémies.

(57) La présente invention concerne l'utilisation du gluconate de zinc pour la fabrication d'un médicament administré par voie orale et comportant de 50 à 150 mg de gluconate de zinc, ledit médicament étant utilisé pour le traitement des hyperprolactinémies.

EP 0 185 586 A2

Médicaments à base de gluconate de zinc utiles pour le traitement des hyperprolactinémies.

La présente invention concerne un nouveau médicament fortement dosé en gluconate de zinc utile pour le traitement des hyperprolactinémies.

On a déjà préconisé l'utilisation de sels de zinc, notamment les acétate, chlorure, sulfate ou gluconate pour le traitement de certaines maladies. C'est ainsi qu'il existe sur le marché des ampoules contenant chacune 0,5 mg environ de sel de zinc, éventuellement associé à des sels de cuivre our éventuellement à des sel de nickel et de cobalt.

On a par ailleurs récemment publié (revue de médecine No. 32, octobre 1982) un article remarquablement documenté faisant le point des connaissances sur le zinc, sa régulation physiologique chez l'homme et les troubles liés à son déficit ou à son excès. Dans cette publication, on suggère la possibilité d'administrer journellement à des patients des sels de zinc à dose de 10 à 45 mg de zinc par jour pour le traitement des états de carence nutritionnelle en zinc et en particulier pour le traitement de l'acrodermatite. Mais, dans la pratique, il semble que l'on ne trouve pas aujourd'hui sur le marché de médicaments permettant l'administration de zinc en hautes quantités.

Il a été trouvé maintenant, et c'est là l'objet de la présente invention, que les médicaments nouveaux, administrés par voie orale, et contenant par dose unitaire de 50 à 150 mg de gluconate de zinc, sont utiles pour le traitement des hyperprolactinémies, telles que les hyperprolactinémies par adénome hypophysaire, les hyperprolactinémies fonctionnelles et les hyperprolactinémies iatrogènes. Dans les médicaments selon l'invention le gluconate de zinc est avantageusement en association avec un véhicule pharmaceutiquement acceptable.

Ces médicaments peuvent être sous la forme de solutions, de comprimés ou de gélules, ces dernières étant la forme de présentation préférée. On indiquera à titre d'exemple qu'une posologie moyenne de 200 mg de gluconate de zinc, per os, en deux prises, convient pour le traitement des hyperprolactinémies.

L'invention est ainsi fondée sur les deux constatations suivantes:

- la remarquable tolérance gastrique - plus généralement sur le plan digestif - du gluconate de zinc, même lorsqu'il est administré à des doses élevées,

- le fait que les hyperprolactinémies metionnées ci-dessus puissent être sensibles à un traitement à base de zinc.

Bien évidemment, l'innocuité du gluconate de zinc aux doses utilisées a été vérifiée chez le rat et chez l'homme.

Le traitement des hyperprolactinémies par le gluconate de zinc à des doses élevées doit être considéré essentiellement comme un traitement de prévention et/ou d'entretien, c'est-à-dire un traitement suivi régulièrement pendant plusieurs mois.

Les exemples suivants illustrent l'utilisation du gluconate de zinc à haute dose.

Exemple 1. Madame L. AIME.

Madame L. AIME présentait une hyperprolactinémie au cours d'un "syndrome de selle vide". Les radiographies de la selle turcique ont montré un agrandissement de la selle. L'examen au Scanner a montré un aspect de selle vide.

1) Le dosage de la prolactine basale sans traitement était de 30 µg/l et s'élevait à 59 µg/l sous test au TRH (α). La malade a alors été soumise à un traitement avec le médicament selon l'invention dans les conditions ci-après : administration pendant 15 jours de 2 gélules à 15 mg de zinc par jour (à 10h et à 16h) sans aucun autre traitement. Ce traitement est dénommé ci-après "traitement L 35".

2) Le dosage de la prolactine après traitement L 35 était de 18 µg/l et s'élevait à 40 µg/l sous le test au TRH. Le TRH est le facteur hypothalamique de libération de la thyréostimuline.

3) L'étude de la fonction gonadotrope a donné les résultats suivants : disparition de la pulsatilité de LH (hormone lutéinisante) sous LHRH (facteur hypothalamique de libération de l'hormone lutéinisante) et diminution de la réponse.

Exemple 2. Madam L. YVETTE

Madame L. Yvette présentait une hyperprolactinémie au cours d'un ‾ syndrome des neuroleptiques. Les radiographies de la selle turcique étaient normales.

1) Le dosage de la prolactine basale sans traitement était de 60 µg/l et s'élevait à 80 µg/l sous TRH.

2) Le dosage de la prolactine après traitement L 35 était de 40 µg/l et s'élevait à 56 µg/l sous TRH.

Exemple 3. Madame P

Madame P. âgée de 45 ans présentait une hyperprolactinémie par microadénome de l'hypophyse connue depuis quatre ans. Les radiographies de la selle turcique ont montré un aspect de double fond.

1) Le dosage de la prolactine basale sans traitement était de 70 µg/l et s'élevait à 93 µg/l au cours du test au TRH.

2) Dosage de la prolactine après traitement L 35 : prolactine à 58 µg/l s'élevant à 64 µg/l au cours du test au TRH.

3) Etude de la fonction gonadotrope: diminution de la pulsatilité de LH sous LHRH.

4) Fonction thyréotrope : pas de modification.

**Revendications**

1. Utilisation du gluconate de zinc pour la fabrication d'un médicament administré par voie orale et comportant de 50 à 150 mg de gluconate de zinc, ledit médicament étant utilisé pour le traitement des hyperprolactinémies.

2. Utilisation selon la revendication 1, caractérisée en ceque le médicament est sous forme de gélule.